(19) **Europäisches Patentamt / European Patent Office / Office européen des brevets**

(11) **EP 4 512 343 A2**

(12) **EUROPEAN PATENT APPLICATION**
published in accordance with Art. 153(4) EPC

(43) Date of publication:
26.02.2025 Bulletin 2025/09

(21) Application number: 23792184.6

(22) Date of filing: 19.04.2023

(51) International Patent Classification (IPC):
*A61B 8/00* (2006.01)  *A61B 8/08* (2006.01)
*G06T 7/00* (2017.01)  *G06N 3/08* (2023.01)

(52) Cooperative Patent Classification (CPC):
A61B 8/00; A61B 8/08; G06N 3/08; G06T 7/00

(86) International application number:
PCT/KR2023/005325

(87) International publication number:
WO 2023/204610 (26.10.2023 Gazette 2023/43)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 19.04.2022  KR 20220048018
18.04.2023  KR 20230051036
18.04.2023  KR 20230051037

(71) Applicant: Ontact Health Co., Ltd.
Seoul 03764 (KR)

(72) Inventors:
• SHIM, Hack Joon
  Seoul 06214 (KR)
• HA, Seong Min
  Seoul 05343 (KR)
• JEONG, Hyun Seok
  Seoul 03725 (KR)
• JEON, Jae Ik
  Uijeongbu-si Gyeonggi-do 11742 (KR)
• CHOI, Annes
  Seoul 04193 (KR)

(74) Representative: Müller Schupfner & Partner
Patent- und Rechtsanwaltspartnerschaft mbB
(Muc)
Bavariaring 11
80336 München (DE)

(54) **ECHOCARDIOGRAPHY GUIDE METHOD AND ECHOCARDIOGRAPHY GUIDE DEVICE USING SAME**

(57) The present invention provides an echocardiography guide method implemented by means of a processor, and a device using same, the echocardiography guide method comprising the steps of: receiving an echocardiographic image of an individual; segmenting a cross section of the echocardiographic image into a plurality of cardiac monolayer regions; calculating, on the basis of a standard cross section received in advance, a matching rate for the cross section from which the plurality of cardiac monolayer regions is segmented; determining, on the basis of the matching rate of the cross section, whether to provide a guide; and, in response to whether to provide the guide, providing the guide for acquiring a cross section having a preset matching rate or higher.

In addition, the present invention provides an echocardiography guide method implemented by means of a processor, and a device using same, the echocardiography guide method comprising the steps of: receiving an echocardiographic image of an individual and a cross section of the image; determining the relative angle of the received cross section with respect to a standard image cross section received in advance; generating, on the basis of the relative angle, a guide for decreasing the relative angle; and providing the guide as a graphic interface.

EP 4 512 343 A2

1000

FIG. 1

## Description

[Technical Field]

The present invention relates to a method for providing a guide for echocardiography measurement and a device for providing a guide for echocardiography using the same.

[Background Art]

An echocardiography examination is performed by a scheme of projecting ultrasound waves on the heart having a three-dimensional structure in multiple-dimensional planes to acquire images of the heart and measure hemodynamic variables.

At this time, a medical staff positions an ultrasound probe in a location where it is easy to acquire ultrasound images, so as to acquire multi-face images through anatomical structures around the heart, such as between the ribs, and records the images by finding an appropriate monolayer through rotation and tilting.

Meanwhile, recently, with the development of technology, a technology has also been proposed which allows integrating a large number of ultrasonic elements to simultaneously acquire a large amount of information and select and view the desired information among them.

However, since an image acquisition process is performed by the medical staff, a long training period can be required to perform an appropriate examination.

Meanwhile, an echocardiography examination process is divided into examination preparation, image acquisition, image analysis, and report writing, and in particular, the image acquisition and analysis process can take a lot of time.

At this time, if an image corresponding to a standard measurement cross section (view) is not acquired during the image analysis process, it may be difficult to diagnose heart disease because measurement values such as an area of a cardiac structure corresponding to the left ventricle, left atrium, right ventricle, right atrium, etc., are not properly measured.

Furthermore, if the image corresponding to a standard measurement cross section (view) is not acquired during the image analysis process, re-examination is inevitable, errors can occur in essential measurement values, and inspection time judgment can be essential depending on each cross section (view).

That is, since a deviation of the acquired ultrasound image can be large depending on the skill level of the medical staff, there is a continuous demand for the development of a guide providing system for an ultrasound wave.

In addition, during the image acquisition process, visualization functions such as two-dimensional image navigation for a process in which the medical staff moves a probe based on a virtual three-dimensional structure of the entire heart can be required.

The background art of the present invention was written to facilitate understanding of the present invention. It should not be understood that the matters described in the background art of the present invention are recognized as prior art.

[Disclosure]

[Technical Problem]

The inventors of the present invention recognized a fact that there is difficulty in acquiring an image corresponding to a standard measurement cross section (view) during an image analysis process.

In particular, the inventors of the present invention provided whether a cross section of an ultrasound image currently acquired through the recognition corresponds to a standard measurement cross section as a matching rate to conceive that the higher the matching rate, the closer the matching rate is to the standard measurement cross section. As a result, the inventors of the present invention could develop a guide system for acquiring a measurement value by segmenting a plurality of regions with respect to an echocardiographic image, calculating a matching rate by comparing the measurement value and the standard measurement cross section, and acquiring the standard measurement cross section based thereon.

The inventors of the present invention could expect that by providing a new guide system, it is possible to overcome the limitation that it is difficult to acquire an accurate measurement value of a cardiac structure in the related art by facilitating image acquisition corresponding to the standard measurement cross section.

In particular, the inventors of the present invention could expect to overcome the limitation of low reliability and being provided for an entry-level medical staff or for an educational purpose because the conventional echocardiography guide system presents a uniform guide for the direction, rotation, angle, etc., of a probe.

In addition, it could be expected that during the image acquisition process, visualization functions such as two-dimensional image navigation for a process in which the medical staff moves a probe based on a virtual three-dimensional structure of the entire heart are provided to provide an intuitive echocardiographic image guide.

Accordingly, an object to be achieved by the present invention is to provide an echocardiography guide method and an echocardiography guide device using an artificial neural network-based guide system configured to present a guideline for acquiring measurement values by segmenting an echocardiographic image into a plurality of regions, calculating a matching rate by comparing the measurement values with a standard measurement cross section (view), and acquiring the standard measurement cross section (view) based on the calculated matching rate.

Furthermore, the inventors of the present invention conceived that when a guide for determining a relative angle between a standard image cross section received in advance and the received cross section by using an Euler angle through the recognition described above, and reducing the relative angle based on the relative angle is generated, and the guide is provided as a graphic interface, the measurement value may be closer to the standard measurement cross section.

As a result, the inventors of the present invention could develop the guide system by generating a guide for determining a relative angle between a standard image cross section received in advance and the received cross section by using an Euler angle, and reducing the relative angle based on the relative angle.

The inventors of the present invention could expect that by providing a new guide system, it is possible to overcome the limitation that it is difficult to acquire an accurate measurement value of a cardiac structure in the related art by facilitating image acquisition corresponding to the standard measurement cross section.

In particular, the inventors of the present invention recognized the need for a guide system applicable to spectral Doppler requiring fine tuning as well as echocardiography B-mode and M-mode.

As a result, the inventors of the present invention were able to develop an artificial neural network-based guide system capable of presenting a guideline by evaluating a cross section matching degree and evaluating the relative angle matching rate to enable fine tuning.

In particular, the inventors of the present invention could expect that it is possible to overcome the limitation of low reliability and being provided for an entry-level medical staff or for an educational purpose because the guide system in the related art presents a guide based on a threshold.

Accordingly, an object to be achieved by the present invention is to provide a guide providing method and a guide providing device configured to receive an echocardiographic image of an individual, classify a cross section for the echocardiographic image, determine a relative angle, and provide a guide based on an evaluation result.

The objects of the present invention are not limited to the aforementioned objects, and other objects, which are not mentioned above, will be apparent to a person having ordinary skill in the art from the following description.

[Technical Solution]

In order to achieve the objects described above, provided is an echocardiography guide method according to an exemplary embodiment of the present invention.

The guide method includes: receiving an echocardiographic image of an individual; segmenting a cross section of the echocardiographic image into a plurality of cardiac monolayer regions; calculating a matching rate for the cross section from which the plurality of cardiac monolayer regions is segmented based on a standard cross section received in advance; determining whether to provide a guide based on the matching rate for the cross section; and providing the guide for acquiring a cross section having a preset matching rate or higher in response to whether to provide the guide.

According to an exemplary embodiment of the present invention, the segmenting of the cross section of the echocardiographic image into the cardiac monolayer regions may further include segmenting each of the plurality of cardiac monolayer regions by using a first prediction model trained to segment the cross section into each of the plurality of cardiac monolayer regions.

According to another exemplary embodiment of the present invention, the calculating of the matching rate for the cross section based on the standard cross section received in advance may further include calculating a matching rate by using a matching rate calculation model configured to calculate the matching rate by outputting measurement values from the segmented cardiac structure regions and comparing the measurement value with a measurement value determined in the cross section based on the standard cross section received in advance.

According to a feature of the present invention, the plurality of cardiac monolayer regions may be any one or more selected from structures observable in a specific cardiac cross section, such as the right ventricle (RV), the left ventricle (LV), the Aorta, the left atrium (LA), the left ventricle posterior wall (LVPW), and the intect ventricular septum (IVS).

According to yet another exemplary embodiment of the present invention, the first prediction model which is trained to segment the cross section into each of the plurality of cardiac monolayer regions may include a first feature extraction unit configured to input a received echocardiographic image and segment a plurality of regions based on the anatomical structure within the echocardiographic image, and a second feature extraction unit configured to input the received echocardiographic image and predict positions of a plurality of points within the echocardiographic image.

According to still yet another exemplary embodiment of the present invention, the first prediction model may be trained to segment the cross section into each of the plurality of cardiac monolayer regions, and may be further configured to evaluate the region segmentation result by inputting an entropy, and mask information including an anatomical mask for each region (inferenced chamber mask anatomy) and an area for each region (inferenced chamber mask ratio) with respect to the segmented regions.

According to still yet another exemplary embodiment of the present invention, the matching rate calculation model may be configured to calculate a measurement value corresponding to an area and a size of each of the segmented cardiac monolayer regions by inputting the plurality of points in the echocardiographic image determined by the first prediction model, and further configured to calculate the matching rate by comparing the measurement value determined from the cross section based on the standard cross section received in advance.

According to still yet another exemplary embodiment of the present invention, the matching rate calculation model may be further configured to calculate the matching rate by evaluating measurement values measured in the segmented cardiac monolayer regions by inputting data from an entropy meter, a capacity meter, and a connectivity meter, and comparing the measurement value determined in the cross section based on the standard cross section received in advance.

According to an exemplary embodiment of the present invention, the guide may include the matching rate calculated by comparing the measurement value determined in the cross section based on the standard cross section received in advance, and movement information of the probe for acquiring the standard measurement cross-section (view).

A visualization function such as a navigation of a two-dimensional image according to a position and a direction in which a probe moves in a virtual three-dimensional structure for the entire heart may be included with respect to movement of the probe.

In order to achieve the objects described above, provided is an echocardiography guide device according to another exemplary embodiment of the present invention. The guide device includes: a communication unit configured to receive an echocardiographic image of an individual; and a processor functionally connected to the communication unit.

The processor is configured to segment the cross section of the echocardiography image into a plurality of cardiac monolayer regions, calculate a matching rate for the cross section in which the plurality of cardiac monolayer regions are segmented based on a previously received standard cross section, and determines whether to provide the guide based on the matching rate for the cross section, and provide a guide for acquiring a cross section having a preset matching rate or higher in response to whether to provide the guide.

According to a feature of the present invention, the processor may be further configured to segment each of the plurality of cardiac monolayer regions using a first prediction model trained to segment the cross section into the plurality of cardiac monolayer regions, respectively.

According to another feature of the present invention, the processor may be further configured to calculate a matching rate by using a matching rate calculation model configured to calculate the matching rate by outputting measurement values from the segmented cardiac structure regions and comparing the measurement value with a measurement value determined in the cross section based on a standard cross section received in advance.

According to a feature of the present invention, the plurality of cardiac monolayer regions may be one or more selected from structures observable in a specific cardiac cross section, such as the right ventricle (RV), the left ventricle (LV), the aorta, the left atrium (LA), the left ventricle posterior wall (LVPW), and the intect ventricular septum (IVS).

According to another feature of the present invention, the first prediction model may be trained to segment the cross section into each of a plurality of cardiac monolayer regions, and may include a first feature extraction unit configured to input a received echocardiographic image and segment a plurality of regions based on the anatomical structure within the echocardiographic image, and a second feature extraction unit configured to input the received echocardiographic image and predict positions of a plurality of points within the echocardiographic image.

According to yet another feature of the present invention, the first prediction model may be trained to segment the cross section into each of the plurality of cardiac monolayer regions, and may be further configured to evaluate the region segmentation result by inputting an entropy, and mask information including an anatomical mask for each region (inferenced chamber mask anatomy) and an area for each region (inferenced chamber mask ratio) with respect to the segmented regions.

According to still yet another feature of the present invention, the matching rate calculation model may be configured to calculate a measurement value corresponding to an area and a size of each of the segmented cardiac monolayer regions by inputting the plurality of points in the echocardiographic image determined by the first prediction model, and further configured to calculate the matching rate by comparing the measurement value determined from the cross section based on the standard cross section received in advance.

According to still yet another feature of the present invention, the matching rate calculation model may be further configured to calculate the matching rate by evaluating measurement values measured in the segmented cardiac monolayer regions by inputting data from an entropy meter, a capacity meter, and a connectivity meter, and comparing the measurement value determined in the cross section based on the standard cross section received in advance.

According to an exemplary embodiment of the present invention, the guide may include the matching rate calculated by comparing the measurement value determined in the cross section based on the standard cross section received in advance, and movement information of the probe for acquiring the standard measurement cross section (view).

The guide method, which is implemented by a processor, includes: receiving an echocardiographic image of an individual and a cross section of the image; determining the relative angle of the received cross section with respect to a standard image cross section received in advance; generating, based on the relative angle, a guide for decreasing the relative angle; and providing the guide as a graphic interface.

According to an exemplary embodiment of the present invention, in the determining of the relative angle, an Euler angle may be used.

Further, according to an exemplary embodiment of the present invention, the provided guide may further include a two-dimensional relative angle for a three-dimensional image determined using the Euler angle.

In addition, according to another exemplary embodiment of the present invention, the provided guide may provide a matching degree between relative angles of the cross section and a standard image.

In order to achieve the objects described above, provided is an echocardiography guide device according to another exemplary embodiment of the present invention. The guide device includes: a communication unit configured to receive an echocardiographic image of an individual and a cross section in the image; and a processor functionally connected to the communication unit, and the processor is configured to classify a cross section for the echocardiographic image, determine a relative angle between a standard image cross section received in advance and the received cross section, generate a guide for reducing the relative angle based on the relative angle, and provide the guide as a graphic interface.

According to an exemplary embodiment of the present invention, the processor may be further configured to determine the relative angle using an Euler angle.

According to another exemplary embodiment of the present invention, the processor may be further configured to provide further include a two-dimensional relative angle for a three-dimensional image determined using the Euler angle.

According to another exemplary embodiment of the present invention, the processor may be further configured to provide a guide that provides a matching degree between the relative angles of the cross section and the standard image.

Details of other exemplary embodiments will be included in the detailed description and the drawings.

[Advantageous Effects]

The present invention may provide a guide system for an echocardiography cross section based on an artificial neural network configured to segment a cross section of the echocardiographic image into a plurality of cardiac monolayer regions, calculate a matching rate for the cross section from which the plurality of cardiac monolayer regions is segmented based on a standard cross section received in advance, determine whether to provide a guide based on the matching rate for the cross section, and provide the guide for acquiring a cross section having a preset matching rate or higher in response to whether to provide the guide to provide a guideline for highly reliable echocardiography measurement.

The present invention may overcome the limitations that it is difficult to acquire an accurate measurement value for a cardiac structure in the related art by providing a new guide system.

In particular, the present invention may overcome the limitation of low reliability and being provided for an entry-level medical staff or for an educational purpose because the conventional echocardiography guide system presents a uniform guide for the direction, rotation, angle, etc., of a probe.

Further, the present invention provides a guide system which determines a relative angle between a standard image cross section received in advance and the received cross section, and generates a guide for reducing the relative angle based on the relative angle to provide the guide as a graphic interface to provide a guideline for highly reliable echocardiography measurement.

The present invention may overcome the limitations of a guide system using a threshold-based cross section (view) classification model in the related art by providing a new guide system.

In particular, the present invention may overcome the limitation of low reliability and being provided for an entry-level medical staff or for an educational purpose because the guide system in the related art presents a guide based on a threshold.

The effects according to the present invention are not limited by the contents exemplified above, and other various effects are included in the present specification.

[Description of Drawings]

FIG. 1 illustrates a guide system for a cross section of an echocardiography using a device for guiding echocardiography according to an exemplary embodiment of the present invention.

FIG. 2A is a block diagram illustrating a configuration of a medical staff device according to an exemplary embodiment of the present invention.

FIG. 2B is a block diagram illustrating a configuration of a guide server according to an exemplary embodiment of the present invention.

FIG. 3 illustrates a procedure of a guide method for echocardiography according to an exemplary embodiment of the present invention.

FIG. 4A is a diagram illustrating an example of a chamber anatomy meter for a result of segmenting a cardiac monolayer region based on a standard cross section received in advance according to an exemplary embodiment of the present invention.

FIG. 4B is a diagram illustrating an example of a ratio meter for the result of segmenting the cardiac monolayer region based on the standard cross section received in advance according to an exemplary embodiment of the present invention.

FIGS. 5A and 5B are diagrams illustrating examples of evaluating the result of segmenting the cardiac monolayer region using an entropy meter according to an exemplary embodiment of the present invention.

FIGS. 6A to 6C are diagrams illustrating examples of evaluating the result of segmenting the cardiac monolayer region using a capacity meter according to an exemplary embodiment of the present invention.

FIG. 7 is a diagram illustrating an example of evaluating the result of segmenting the cardiac monolayer region using a connectivity meter according to an exemplary embodiment of the present invention.

FIGS. 8 to 10 are diagrams illustrating examples of a guideline presented by using a guide method for echocardiography according to an exemplary embodiment of the present invention.

FIG. 11 illustrates a guide system for a cross section of an echocardiography using an echocardiography guide device according to an exemplary embodiment of the present invention.

FIG. 12A is a block diagram illustrating a configuration of a medical staff device according to an exemplary embodiment of the present invention.

FIG. 12B is a block diagram illustrating a configuration of a guide server according to an exemplary embodiment of the present invention.

FIG. 13 illustrates a procedure of an echocardiography guide method according to an exemplary embodiment of the present invention.

FIG. 14 is an exemplary diagram illustrating determining a relative angle using an Euler angle according to an exemplary embodiment of the present invention.

FIG. 15 illustrates a procedure for reducing a relative angle according to various exemplary embodiments of the present invention.

[Best Mode]

Advantages of the present invention, and methods for accomplishing the same will be more clearly understood from exemplary embodiments described in detail below with reference to the accompanying drawings. However, the present invention is not limited to the following exemplary embodiments but may be implemented in various different forms. The exemplary embodiments are provided only to make description of the present invention complete and to fully provide the scope of the present invention to a person having ordinary skill in the art to which the present invention pertains.

The shapes, sizes, ratios, angles, numbers, and the like illustrated in the drawings for describing the exemplary embodiments of the present invention are merely examples, and the present invention is not limited thereto. Further, in describing the present invention, a detailed explanation of known related technologies may be omitted to avoid unnecessarily obscuring the subject matter of the present invention. The terms such as "including," "having," and "consisting of" used herein are intended to allow other components to be added unless the terms are used with the term "only". Any references in a case where a component is expressed as a singular, the case includes the plural unless expressly stated otherwise.

Components are interpreted to include an error range even if not expressly stated.

The features of various exemplary embodiments of the present invention can be partially or entirely bonded to or combined with each other and can be interlocked and operated in technically various ways to be sufficiently appreciated by those skilled in the art, and the exemplary embodiments can be carried out independently of or in association with each other.

In this specification, a 'standard image' may mean an image corresponding to a standard measurement cross section (view).

Hereinafter, various exemplary embodiments of the present invention will be described in detail with reference to the accompanying drawings.

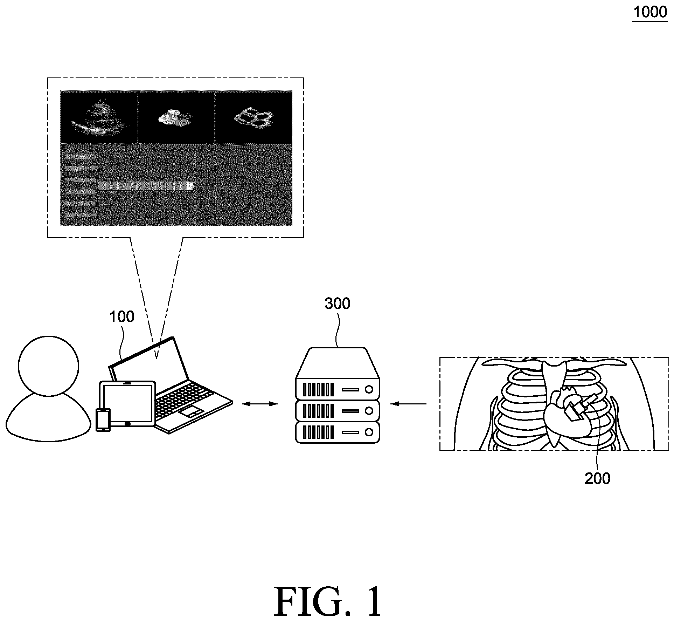

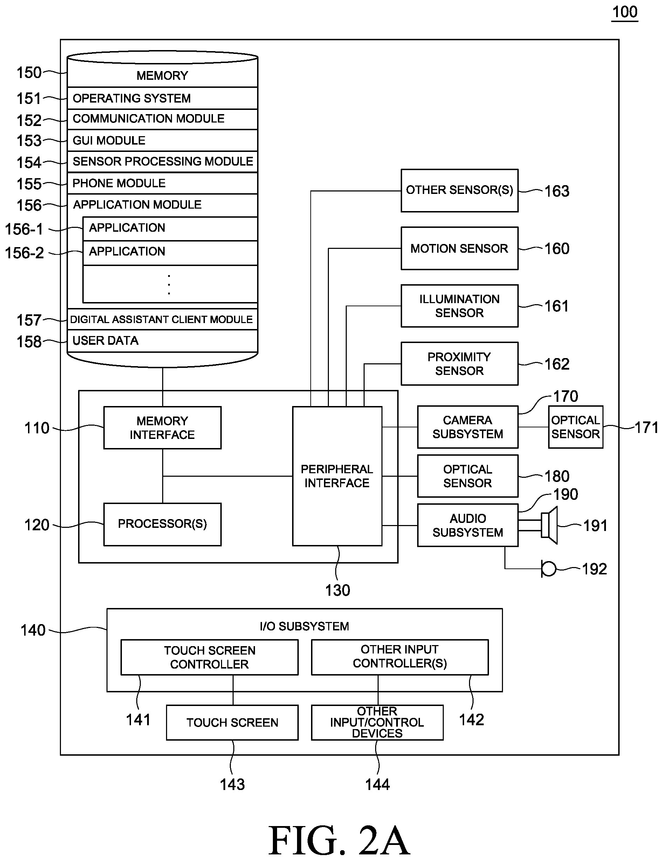

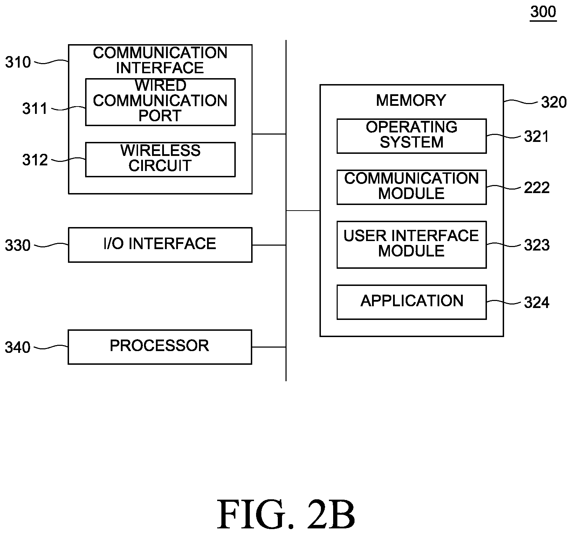

Hereinafter, with reference to FIGS. 1, 2A, and 2B, a guide system for a cross section (view) of an echocardiography using an echocardiography guide device and an echocardiography guide device according to an exemplary embodiment of the present invention will be described.

FIG. 1 illustrates a guide system for a cross section of an echocardiography using an echocardiography guide device according to an exemplary embodiment of the present invention.

FIG. 2A is a block diagram illustrating a configuration of a medical staff device according to an exemplary embodiment of the present invention.

FIG. 2B is a block diagram illustrating a configuration of a guide server according to an exemplary embodiment of the present invention.

First, referring to FIG. 1, the guide system 1000 may be a system configured to provide a guide for echocardiography measurement based on an echocardiographic image of an individual. In this case, the guide system 1000 may be constituted by a medical staff device 100 receiving a guide for echocardiography measurement, an echocardiographic image diagnosis device 200 providing the echocardiographic image, and a guide server 300 generating the guide for echocardiography measurement based on the received echocardiographic image.

First, next, the medical staff device 100 as an electronic device providing a user interface for representing the guide for echocardiography measurement may include at least one of a smartphone, a tablet personal computer (PC), a laptop computer, and/or a PC.

The medical staff device 100 may receive prediction results associated with a matching rate with an electrocardiography cross section for the individual and a standard cross section from the guide server 300, and display the received results through a display unit to be described below.

The guide server 300 may include a general-purpose computer, laptop, and/or data server that perform various operations for determining the guide for echocardiography measurement based on the echocardiographic image provided from the echocardiographic image diagnosis device 200 such as an ultrasound diagnosis device. At this time, the guide server 300 may be a device for accessing a web server providing a web page or a mobile web server providing a mobile website, but is not limited thereto.

More specifically, the guide server 300 receives the echocardiographic image of the individual from the echocardiographic image diagnosis device 200, segments the cross section of the echocardiographic image into a plurality of cardiac monolayer regions, calculates a matching rate for the cross section in which the plurality of cardiac monolayer regions is segmented based on a standard cross section received in advance, and determines whether to provide the guide based on the matching rate for the cross section to provide a guide for acquiring a cross section (view) having a preset matching rate or higher in response to whether to provide the guide.

The guide server 300 may provide the prediction results to the medical staff device 100.

Information provided from the guide server 300 may be provided to a web page through a web browser installed in the medical staff device 100, or provided in the form of an application or a program. In various exemplary embodiments, the data may be provided in a form that is embedded in a platform in a client-server environment.

Next, with reference to FIGS. 2A and 2B, components of the guide server 300 of the present invention will be described in detail.

First, referring to FIG. 2A, the medical staff device 100 may include a memory interface 110, one or more processors 120, and a peripheral interface 130. Various components within the medical staff device 100 may be connected by one or more communication buses or signal lines.

The memory interface 110 is connected to the memory 150 to transmit various data to the processor 120. Here, the memory 150 may include at least one type of storage medium among flash memory type, hard disk type, multimedia card micro type, card type memory (for example, SD or XD memory, etc.), RAM, SRAM, ROM, EEPROM, PROM, network storage, cloud, and blockchain data.

In various exemplary embodiments, the memory 150 may store at least one of an operating system 151, a communication module 152, a graphical user interface module (GUI) 153, a sensor processing module 154, a phone module 155, and an application module 156. Specifically, the operating system 151 may include instructions for processing basic system services and instructions for performing hardware tasks. The communication module 152 may communicate with at least one of one or more other devices, computers, and servers. The graphical user interface module (GUI) 153 may process a graphical user interface. The sensor processing module 154 may process sensor-related functions (e.g., processing a voice input received using one or more microphones 192). The phone module 155 may process phone-related functions. The application module 156 may perform various functions of the user application, such as electronic messaging, web browsing, media processing, navigation, imaging, and other processing functions. Moreover, the medical staff device 100 may store one or more software applications 156-1 and 156-2 (e.g., a guide application) associated with any one type of service in the memory 150.

In various exemplary embodiments, the memory 150 may store a digital assistant client module (157) (hereinafter, referred to as DA client module), and thus store instructions for performing client-side functions of a digital assistant and various user data 158.

Meanwhile, the DA client module 157 may acquire the user's voice input, text input, touch input, and/or gesture input through various user interfaces (e.g., I/O subsystem 140) provided in the medical staff device 100.

Further, the DA client module 157 may output data in audiovisual and tactile forms. For example, the DA client module 157 may output data consisting of a combination of at least two of voice, sound, notification, text message, menu, graphic, video, animation, and vibration. Moreover, the DA client module 157 may communicate with a digital assistant server (not illustrated) using a communication subsystem 180.

In various exemplary embodiments, the DA client module 157 may collect additional information about a surrounding environment of the medical staff device 100 from various sensors, subsystems, and peripheral devices to construct a context associated with the user input. For example, the DA client module 157 may infer a user's intention by providing context information to a digital assistance server jointly with the user input. Here, the context information that may accompany the user input may include sensor information, such as lighting, ambient noise, ambient temperature, images of the surrounding environment, video, etc. As another example, the context information may include a physical state of the medical staff device 100 (e.g., device orientation, device position, device temperature, power level, speed, acceleration, motion pattern, cellular signal strength, etc.). As yet another example, the context information may include information related to the software state of the medical staff device 100 (e.g., processes running on the medical staff device 100, installed programs, past and current network activity, background services, error logs, resource usage, etc.).

In various exemplary embodiments, the memory 150 may include additional or deleted instructions, and may further include additional components other than those illustrated in FIG. 2A of the medical staff device 100, or may exclude some components.

The processor 120 may control the overall operation of the medical staff device 100 and execute various instructions to implement an interface that provides the guide for echocardiography measurement by running an application or program stored in the memory 150.

The processor 120 may correspond to an operation device such as a central processing unit (CPU) or an application processor (AP). Further, the processor 120 may be implemented in the form of an integrated chip (IC) such as a system on chip (SoC) in which various operation devices such as a neural processing unit (NPU) are integrated.

The peripheral interface 130 is connected to various sensors, subsystems, and peripheral devices to provide data so that the medical staff device 100 may perform various functions. Here, it may be understood that any function performed by the medical staff device 100 is performed by the processor 120.

The peripheral interface 130 may receive data from a motion sensor 160, an illumination sensor (optical sensor) 161, and a proximity sensor 162, and through this, the medical staff device 100 may perform orientation, light, and proximity detection functions. As another example, the peripheral interface 130 may receive data from other sensors 163 (positioning system - GPS receiver, temperature sensor, biometric sensor), thereby enabling the medical staff device 100 to perform functions related to the other sensors 163.

In various exemplary embodiments, the medical staff device 100 may include a camera subsystem 170 connected to the peripheral interface 130, and an optical sensor 171 connected thereto, which may enable the medical staff device 100 to perform various photographing functions, such as taking photographs and recording video clips.

In various exemplary embodiments, the medical staff device 100 may include a communication subsystem 180 connected to the peripheral interface 130. The communication subsystem 180 consists of one or more wired/wireless networks and may include various communication ports, radio frequency transceivers, and optical transceivers.

In various exemplary embodiments, the medical staff device 100 includes an audio subsystem 190 connected to the peripheral interface 130, the audio subsystem 190 includes one or more speakers 191 and one or more microphones 192, such that the medical staff device 100 may perform voice-activated functions, such as speech recognition, voice replication, digital recording, and telephony functions.

In various exemplary embodiments, the medical staff device 100 may include an I/O subsystem 140 connected to the peripheral interface 130. For example, the I/O subsystem 140 may control a touch screen 143 included in the medical staff device 100 via a touch screen controller 141. As an example, the touch screen controller 141 may detect a user's contact and movement or cessation of contact and movement using any one of a plurality of touch sensing technologies, such as capacitive, resistive, infrared, surface acoustic wave technology, proximity sensor array, etc. As another example, the I/O subsystem 140 may control other input/control devices 144 included in the medical staff device 100 via other input controller(s) 142. As an example, the other input controller(s) 142 may control one or more buttons, rocker switches, thumb-wheels, infrared ports, USB ports, and pointer devices such as a stylus.

Next, referring to FIG. 2B, the guide server 300 may include a communication interface 310, a memory 320, an I/O interface 330, and a processor 340, and respective components may communicate with each other through one or more communication buses or signal lines.

The communication interface 310 may be connected to the medical staff device 100 and the echocardiographic image diagnosis device 200 via a wired/wireless communication network to exchange data. For example, the communication interface 310 may receive the echocardiographic image from the echocardiographic image diagnosis device 200, and transmit determined information to the medical staff device 100.

Meanwhile, the communication interface 310 that enables transmission and reception of such data may include a communication port 311 and a wireless circuit 312, and here, the wired communication port 311 may include one or more wired interfaces, for example, Ethernet, universal serial bus (USB), FireWire, etc. Further, the wireless circuit 312 may transmit and receive data with an external device via RF signals or optical signals. Moreover, the wireless communication may use at least one of a plurality of communication standards, protocols and technologies, such as GSM, EDGE, CDMA, TDMA, Bluetooth, Wi-Fi, VoIP, Wi-MAX, or any other suitable communication protocol.

The memory 320 may store various data used in the guide server 300. For example, the memory 320 may store the echocardiographic image, store a model trained to classify and segment the cross section for the echocardiographic image, or store models trained to compare and evaluate a measurement value acquired from the echocardiography cross section based on a previously received standard cross section, and calculate a matching rate.

In various exemplary embodiments, the memory 320 may include a volatile or nonvolatile storage medium capable of storing various data, instructions, and information. For example, the memory 320 may include at least one type of storage medium among flash memory type, hard disk type, multimedia card micro type, card type memory (for example, SD or XD memory, etc.), RAM, SRAM, ROM, EEPROM, PROM, network storage, cloud, and blockchain data.

In various exemplary embodiments, the memory 320 may store at least one component of an operating system 321, a communication module 322, a user interface module 323, and one or more applications 324.

The operating system 321 (e.g., an embedded operating system such as LINUX, UNIX, MAC OS, WINDOWS, VxWorks, etc.) may include various software components and drivers for controlling and managing general system tasks (e.g., memory management, storage device control, power management, etc.) and may support communication between various hardware, firmware, and software components.

The communication module 323 may support communication with other devices through the communication interface 310. The communication module 320 may include various software components for processing data received by the wired communication port 311 or wireless circuit 312 of the communication interface 310.

The user interface module 323 may receive a user's request or input from a keyboard, touch screen, microphone, etc., through the I/O interface 330 and provide a user interface on a display.

The application 324 may include a program or module configured to be executed by one or more processors 330. Here, the application for providing the guide for echocardiography measurement may be implemented on a server farm.

The I/O interface 330 may connect at least one of an input/output device (not illustrated) of the guide server 300, such as a display, a keyboard, a touch screen, and a microphone, to the user interface module 323. The I/O interface 330 may receive a user input (e.g., a voice input, a keyboard input, a touch input, etc.) together with the user interface module 323, and process an instruction according to the received input.

The processor 340 is connected to the communication interface 310, the memory 320, and the I/O interface 330 to control the overall operation of the guide server 300, and may perform various instructions for the guide through the application or program stored in the memory 320.

The processor 340 may correspond to an operation device such as a central processing unit (CPU) or an application processor (AP). Further, the processor 340 may be implemented in the form of an integrated chip (IC) such as a system on chip (SoC) in which various operation devices are integrated. Alternatively, the processor 340 may include a module for calculating an artificial neural network model, such as a neural processing unit (NPU).

In various exemplary embodiments, the processor 340 may be configured to receive the echocardiographic image of the individual, segment the cross section of the echocardiographic image into a plurality of cardiac monolayer regions, calculate a matching rate for the cross section in which the plurality of cardiac monolayer regions is segmented based on a standard cross section received in advance, and determines whether to provide the guide based on the matching rate for the cross section, and provide a guide for acquiring a cross section having a preset matching rate or higher in response to whether to provide the guide. Optionally, the processor 340 may be configured to provide a measurement guide for each echocardiography cross section.

In various exemplary embodiments, the processor 340 may be further configured to segment the plurality of cardiac monolayer regions using a first prediction model trained to segment the plurality of cardiac monolayer regions in the cross section, respectively.

In various exemplary embodiments, the processor 340 may be further configured to calculate a matching rate by using a matching rate calculation model configured to calculate the matching rate by outputting measurement values from the segmented cardiac structure regions and comparing the measurement value with a measurement value determined in the cross section based on a standard cross section received in advance.

At this time, the first prediction model may be trained to segment each of a plurality of cardiac monolayer regions in the cross section, and may include a first feature extraction unit configured to input a received echocardiographic image and segment a plurality of regions based on the anatomical structure within the echocardiographic image, and a second feature extraction unit configured to input the received echocardiographic image and predict positions of a plurality of points within the echocardiographic image.

At this time, the first prediction model may be trained to segment the cross section into each of the plurality of cardiac monolayer regions, and may be further configured to evaluate the region segmentation result by inputting an entropy, and mask information including an anatomical mask for each region (inferenced chamber mask anatomy) and an area for each region (inferenced chamber mask ratio) with respect to the segmented regions.

At this time, the matching rate calculation model may be configured to calculate a measurement value corresponding to an area and a size of each of the segmented cardiac monolayer regions by inputting the plurality of points in the echocardiographic image determined by the first prediction model, and may be further configured to calculate the matching rate by comparing the measurement value determined in the cross section based on the standard cross section received in advance.

At this time, the matching rate calculation model may be further configured to evaluate measurement values measured in the segmented cardiac monolayer regions by inputting data from an entropy meter, a capacity meter, and a connectivity meter, and calculate the matching rate by comparing the measurement value determined in the cross section based on the standard cross section received in advance.

In various exemplary embodiments, the processor 340 may be further configured to provide a guide including the matching rate calculated by comparing the measurement value determined in the cross section based on the standard cross section received in advance, and movement information of the probe for acquiring the standard measurement cross section (view).

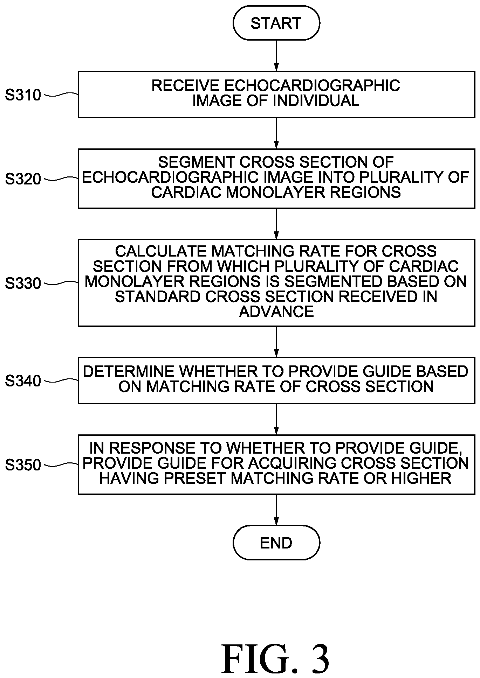

Hereinafter, an echocardiography guide method according to an exemplary embodiment of the present invention will be described in detail with reference to FIG. 3.

FIG. 3 illustrates a procedure of an echocardiography guide method according to an exemplary embodiment of the present invention.

First, the guide procedure according to an exemplary embodiment of the present invention will be described below with reference to FIG. 3.

Provided is a guide method including a step of receiving the echocardiographic image of the individual, a step of segmenting the cross section of the echocardiographic image into a plurality of cardiac monolayer regions, a step of calculating a matching rate for the cross section in which the plurality of cardiac monolayer regions is segmented based on a standard cross section received in advance, a step of determining whether to provide the guide based on the matching rate for the cross section, and a step of providing a guide for acquiring a cross section having a preset matching rate or higher in response to whether to provide the guide.

At this time, the step of segmenting the monolayer region in the cross section may further include a step of segmenting the plurality of cardiac monolayer regions using the first prediction model trained to segment the plurality of cardiac monolayer regions in the cross section, respectively.

In addition, the step of calculating the matching rate for the cross section based on the standard cross section received in advance may further include a step of calculating the matching rate by using the matching rate calculation model configured by outputting measurement values in the segmented cardiac structure regions and comparing the measurement values with the measurement value determined in the cross section based on the standard cross section received in advance.

In an exemplary embodiment of the present invention, the plurality of cardiac monolayer regions may be at least one selected from structures observable in a specific cross section of the heart, such as the right ventricle (RV), the left ventricle (LV), the aorta (Aorta), the left atrium (LA), the left ventricle posterior wall (LVPW), and the intect ventricular septum (IVS).

In various exemplary embodiments of the present invention, the first prediction model may be trained to segment a plurality of cardiac monolayer regions in the cross section, respectively, and may include a first feature extraction unit configured to input a received echocardiographic image and segment a plurality of regions based on the anatomical structure within the echocardiographic image, and a second feature extraction unit configured to input the received echocardiographic image and predict positions of a plurality of points within the echocardiographic image.

At this time, the first prediction model may be trained to segment the cross section into each of the plurality of cardiac monolayer regions, and may be further configured to evaluate the region segmentation result by inputting an entropy, and mask information including an anatomical mask for each region (inferenced chamber mask anatomy) and an area for each region (inferenced chamber mask ratio) with respect to the segmented regions.

In various exemplary embodiments of the present invention, the matching rate calculation model may be configured to calculate a measurement value corresponding to an area and a size of each of the segmented cardiac monolayer regions by inputting the plurality of points in the echocardiographic image determined by the first prediction model, and may be further configured to calculate the matching rate by comparing the measurement value determined in the cross section based on the standard cross section received in advance.

At this time, the matching rate calculation model may be further configured to calculate the matching rate by evaluating measurement values measured in segmented cardiac monolayer regions by inputting data of an entropy meter, a capacity meter, and a connectivity meter by using a measurement value corresponding to an area and a size of each of the segmented cardiac monolayer regions by inputting the plurality of points in the echocardiographic image determined by the first prediction model, and comparing the measurement value determined in the cross section based on the standard cross section received in advance.

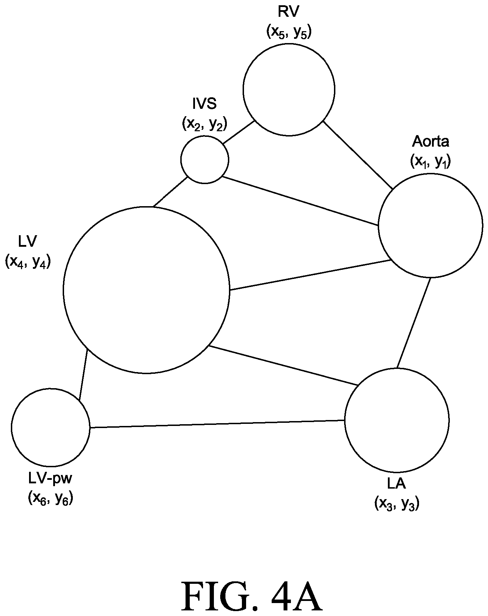

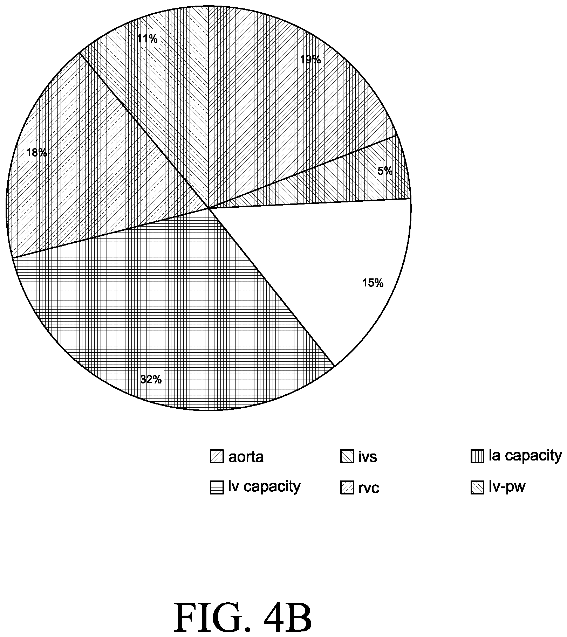

Hereinafter, the echocardiography guide method according to an exemplary embodiment of the present invention will be described in detail with reference to FIGS. 4A and 4B.

According to the present invention, the region segmentation result is evaluated by inputting an entropy, and mask information including an anatomical mask for each region (inferenced chamber mask anatomy) and an area for each region (inferenced chamber mask ratio) to provide an evaluation result with respect to the segmented regions.

FIG. 4A is a diagram illustrating an example of a chamber anatomy meter for a result of segmenting a cardiac monolayer region based on a standard cross section received in advance according to an exemplary embodiment of the present invention.

FIG. 4B is a diagram illustrating an example of a ratio meter for the result of segmenting the cardiac monolayer region based on the standard cross section received in advance according to an exemplary embodiment of the present invention.

Referring to FIG. 4A, the chamber anatomy meter for the result of segmenting the cardiac monolayer region based on the standard cross section received in advance according to an exemplary embodiment of the present invention may be previously predicted in advance to probabilistically distribute coordinates of respective points corresponding to the center of each region of the aorta (Aorta), the intect ventricular septum (IVS), the left atrium (LA), the left ventricle (LV), the right ventricle (RV), and the left ventricle posterior wall (LVPW) by inputting the standard measurement cross section received in advance, and for example, in respect to the coordinates at which the respective points may be located, a point corresponding to a center of the Aorta may correspond to $(X_1, Y_1)$, a point corresponding to a center of the intect ventricular septum (IVS) may correspond to $(X_2, Y_2)$, a point corresponding to a center of the left atrium (LA) may correspond to $(X_3, Y_3)$, a point corresponding to a center of the left ventricle (LV) may correspond to $(X_4, Y_4)$, a point corresponding to a center of the right ventricle (RV) may correspond to $(X_5, Y_5)$, and a point corresponding to a center of the left ventricle posterior wall (LVPW) may correspond to $(X_6, Y_6)$. In an exemplary embodiment, the cardiac cross section may include cross sections that may be photographed through a parasternal window, an apical window, a subcostal window, and a suprasternal notch window.

Referring to FIG. 4B, area ratios of each of the plurality of cardiac monolayer regions segmented in the cross section may be measured in advance as 18% for the right ventricle (RV), 32% for the left ventricle (LV), 19% for the aorta, 19% for the left atrium (LA), 11% for the left ventricle posterior wall (LVPW), and 5% for the intect ventricular septum (IVS) by inputting the standard measurement cross section received in advance.

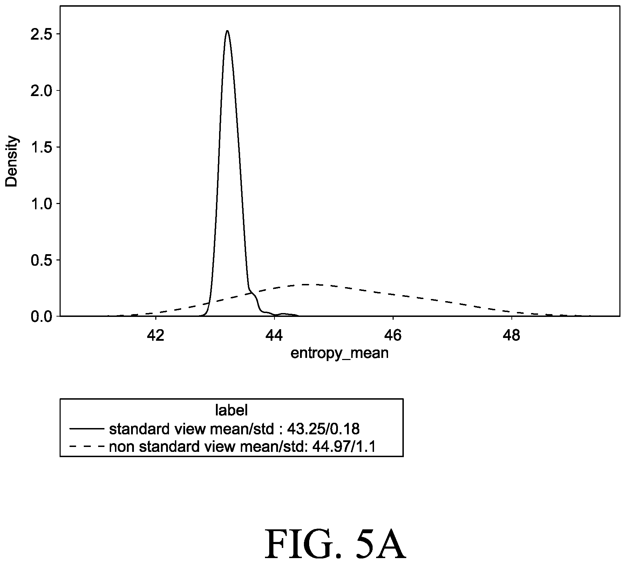

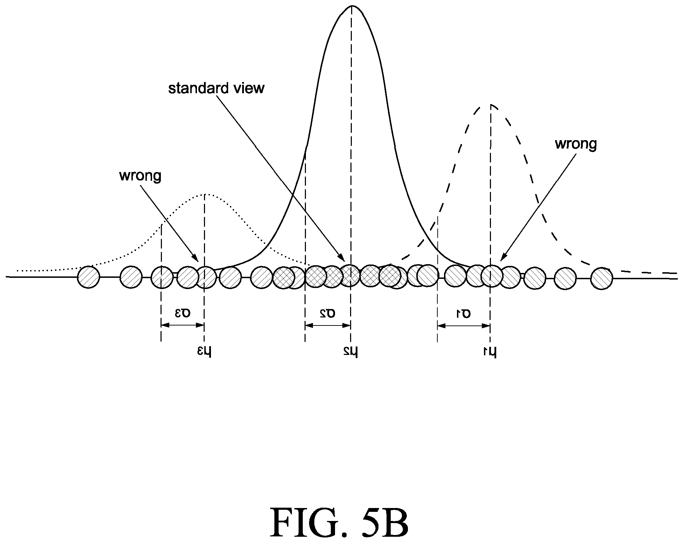

FIGS. 5A and 5B are diagrams illustrating examples of evaluating the result of segmenting the cardiac monolayer region using an entropy meter according to an exemplary embodiment of the present invention.

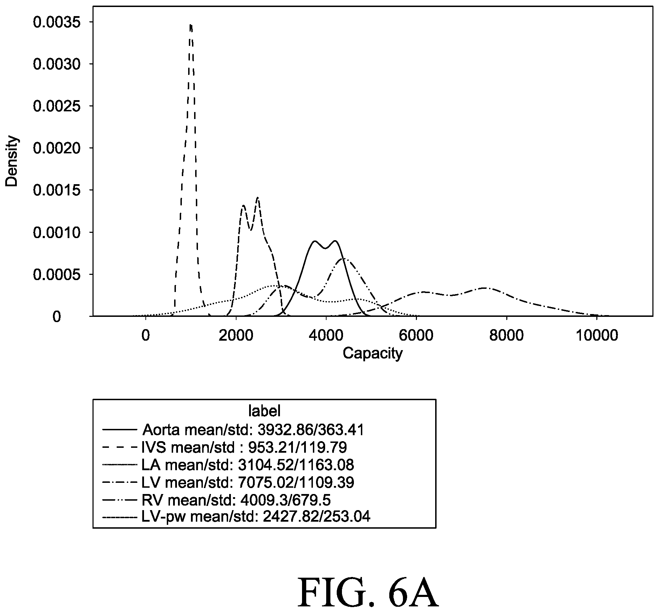

FIGS. 6A to 6C are diagrams illustrating examples of evaluating the result of segmenting the cardiac monolayer region using a capacity meter according to an exemplary embodiment of the present invention.

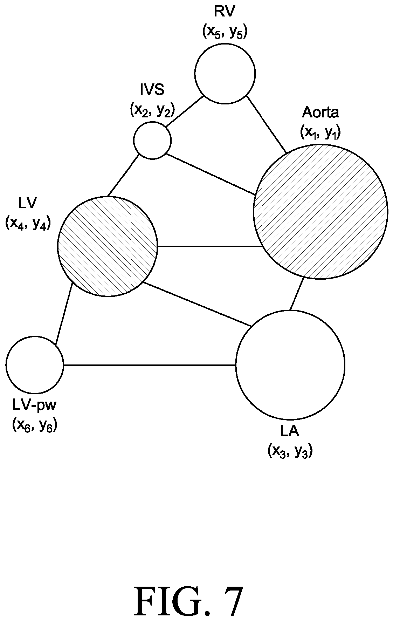

FIG. 7 is a diagram illustrating an example of evaluating the result of segmenting the cardiac monolayer region using a connectivity meter according to an exemplary embodiment of the present invention.

According to an exemplary embodiment of the present invention, the matching rate calculation model of the present invention may be configured to calculate a measurement value corresponding to an area and a size of each of the segmented cardiac monolayer regions by inputting the plurality of points in the echocardiographic image determined by the first prediction model, and may be further configured to calculate the matching rate by comparing the measurement value determined in the cross section based on the standard cross section received in advance.

At this time, the matching rate calculation model may be configured to calculate the matching rate by evaluating measurement values measured in segmented cardiac monolayer regions by inputting data of an entropy meter, a capacity meter, and a connectivity meter by using a measurement value corresponding to an area and a size of each of the segmented cardiac monolayer regions by inputting the plurality of points in the echocardiographic image, and comparing the measurement value determined from the cross section based on the standard cross section received in advance.

First, referring to FIGS. 5A and 5B, FIGS. 5A and 5B illustrate examples of evaluating the results of segmenting the cardiac monolayer region according to an exemplary embodiment of the present invention using an entropy meter, and by inputting data such as an entropy distribution in the standard measurement cross section (standard image, view), an entropy in a plurality of segmented cardiac monolayer regions acquired from a real-time ultrasound image is measured and compared with the entropy distribution in the standard measurement cross section (standard image, view), so it is possible to provide a guideline for probe manipulation for acquiring a cross section such as the standard measurement cross section (standard image, view).

At this time, the entropy may be obtained as in Equations 1 and 2 below.

[Equation 1]

$$Entropy = p \log p$$

[Equation 2]

$$E_{xt}^{'(h,w)} = - \frac{1}{\log(C)} \sum_{c=1}^{C} P_{xt}^{(c,h,w)} \log(P_{xt}^{(c,h,w)})$$

In Equation 1 above, p may represent uncertainty, and in Equation 2 above, $x_t$ may represent a pixel, c may represent a classification, h may represent a height, w may represent a width, and p may represent a probability.

Referring back to FIGS. 5A and 5B, it is possible to compare the entropy of the standard cross section received in advance and the entropy calculated from the cross section in which the plurality of cardiac monolayer regions is segmented based on the entropy meter as described above.

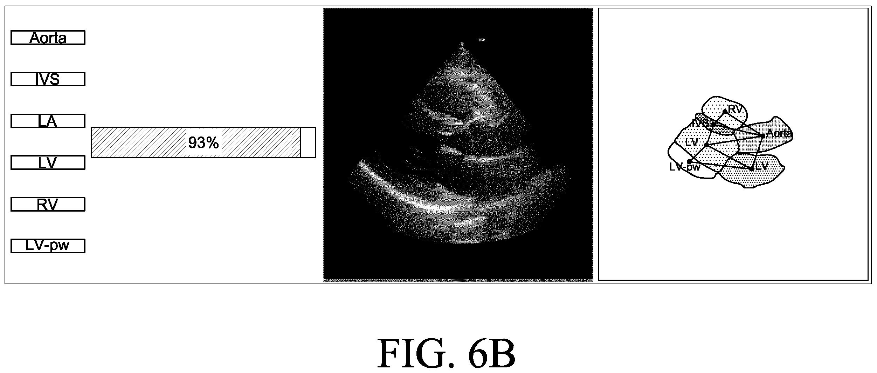

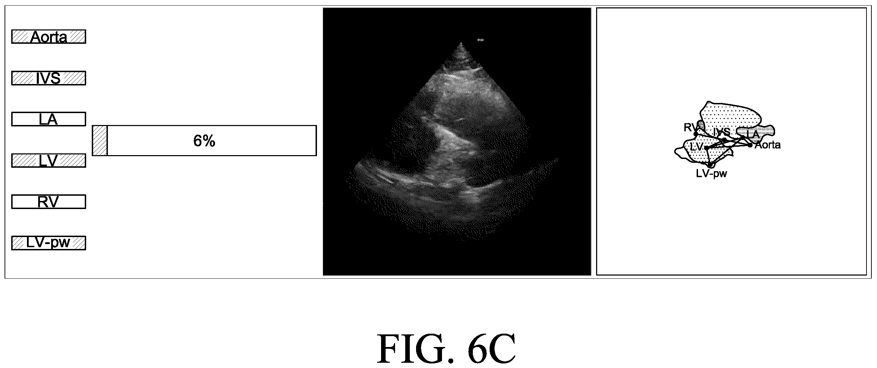

Next, referring to FIG. 6A, FIG. 6A is a diagram illustrating the result of segmenting the cardiac monolayer region, and an example of measuring the capacity of each segmented heart region in the previously received standard cross section according to an exemplary embodiment of the present invention, and FIGS. 6B and 6C are diagrams illustrating examples of evaluating the result of segmenting the cardiac monolayer region by using the connectivity meter according to an exemplary embodiment of the present invention.

Referring back to FIGS. 4A and 4B jointly, coordinates of a plurality of points in the echocardiographic image may be predicted in advance by inputting the standard measurement cross section received in advance, and referring back to FIG. 4B, area ratios of the plurality of respective cardiac monolayer regions segmented in the cross section may be predicted in advance as 18% for the right ventricle (RV), 32% for the left ventricle (LV), 19% for the Aorta, 19% for the left atrium (LA), 11% for the left ventricle posterior wall (LVPW), and 5% for the intect ventricular septum (IVS), so that it is possible to provide the movement guide of the probe for acquiring the standard measurement cross section by comparing the coordinates of the plurality of points and the area of each region measured in each of the segmented regions with the coordinates of the points and the area of each region in the standard measurement cross section received in advance.

Specifically, referring to FIG. 6B, since matching rates with capacities of respective regions which are predicted in advance as 18% for the right ventricle (RV), 32% for the left ventricle (LV), 19% for the Aorta, 19% for the left atrium (LA), 11% for the left ventricle posterior wall (LVPW), and 5% for the intect ventricular septum (IVS) by inputting the standard measurement cross section received in advance is 93%, it may be seen that a capacity matching rate of 90% or more with the standard measurement cross section received in advance is shown.

Referring to FIG. 6C, when the capacities of respective regions which are predicted in advance as 18% for the right ventricle (RV), 32% for the left ventricle (LV), 19% for the Aorta, 19% for the left atrium (LA), 11% for the left ventricle posterior wall (LVPW), and 5% for the intect ventricular septum (IVS) are compared and evaluated by inputting the standard measurement cross section received in advance, it can be seen that when the position coordinates of a plurality of points in the echocardiographic image are also compared with the coordinates which are predicted in advance to be probabilistically distributed by inputting the standard measurement cross section received in advance, the matching rate is just 6%, so the probe should be moved in order to acquire a preset matching rate in the cross section of FIG. 6C.

Next, referring to FIG. 7, FIG. 7 is a diagram illustrating an example of evaluating the result of segmenting the cardiac monolayer region using the connectivity meter according to an exemplary embodiment of the present invention.

Referring back to FIG. 4A, coordinates of respective points corresponding to the center of each region of the aorta, the intect ventricular septum (IVS), the left atrium (LA), the left ventricle (LV), the right ventricle (RV), and the left ventricle posterior wall (LVPW) may be predicted in advance to be probabilistically distributed by inputting the standard measurement cross section received in advance, and for example, in respect to the coordinates at which the respective points may be located, a point corresponding to a center of the aorta may correspond to $(X_1, Y_1)$, a point corresponding to a center of the intect ventricular septum (IVS) may correspond to $(X_2, Y_2)$, a point corresponding to a center of the left atrium (LA) may correspond to $(X_3, Y_3)$, a point corresponding to a center of the left ventricle (LV) may correspond to $(X_4, Y_4)$, a point corresponding to a center of the right ventricle (RV) may correspond to $(X_5, Y_5)$, and a point corresponding to a center of the left ventricle posterior wall (LVPW) may correspond to $(X_6, Y_6)$.

Referring to FIG. 7, according to the present invention, the matching rate may be calculated by comparing the connectivity of each point corresponding to the center of each region with that in the standard measurement cross section received in advance, and in FIG. 7, since it may be confirmed that the point corresponding to the center of the Aorta is enlarged compared to the point corresponding to the center of the Aorta in FIG. 4A, it can be seen that the probe should be moved to the left to acquire a point close to FIG. 4A.

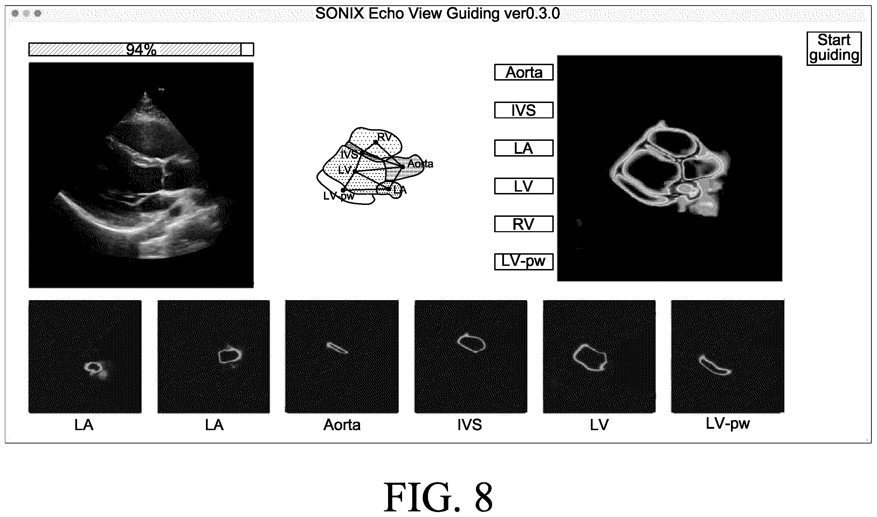

Hereinafter, a guideline presented by utilizing the echocardiography guide method according to various exemplary embodiments of the present invention will be described with reference to FIGS. 8 to 10.

First, referring to FIG. 8, a real-time echocardiographic image is received, the cross section is segmented into each of a plurality of cardiac monolayer regions, and the received echocardiographic image is input to predict the positions of a plurality of points within the echocardiographic image, thereby providing a movement guide of the probe for acquiring the standard measurement cross section.

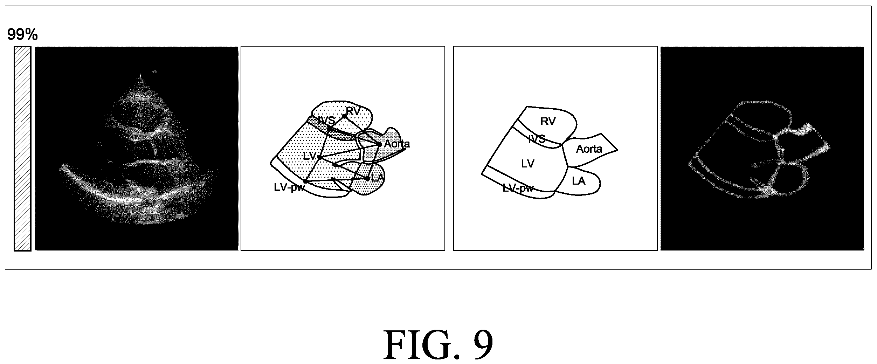

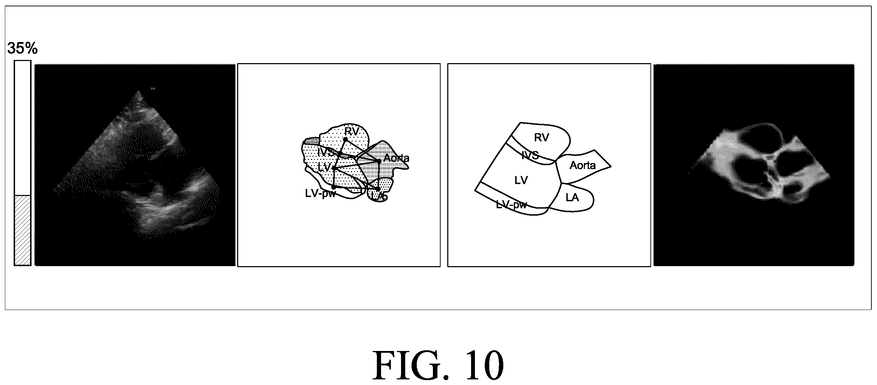

Referring to FIG. 9, FIG. 9 illustrates that the cross section is segmented into a plurality of cardiac monolayer regions in the standard measurement cross section, and the positions and entropy of a plurality of points are measured in each region, and referring to FIG. 10, FIG. 10 is a diagram illustrating a result of segmenting a cardiac monolayer region when the probe acquires the echocardiographic image at a wrong position, and the positions and entropy of the plurality of points measured.

Referring to FIGS. 9 and 10, the segmentation result of the cardiac monolayer region and a plurality of predicted point measurement values are compared with the measurement values determined in the cross section based on the standard cross section received in advance by inputting data of an entropy meter, a capacity meter, and a connectivity meter, and evaluated, and a matching rate with the standard cross section may be calculated based thereon.

Referring to FIG. 9, FIG. 9 indicates a case where a matching rate is 99%, and it is possible to acquire a cross section close to the standard cross section, and referring to FIG. 10, FIG. 10 illustrates a result that the matching rate is calculated as 35%, and in the present invention, as the matching rate in the real-time echocardiographic image may be confirmed, the probe may be enabled to be guided to a clinically meaningful position.

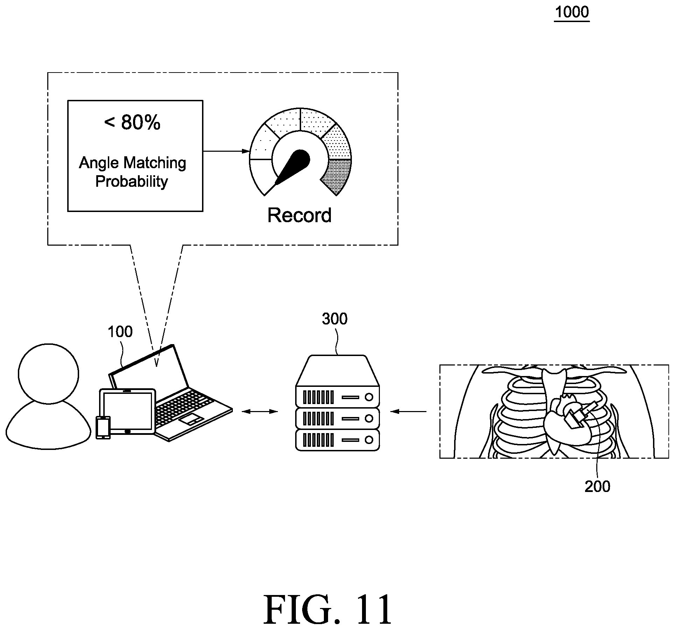

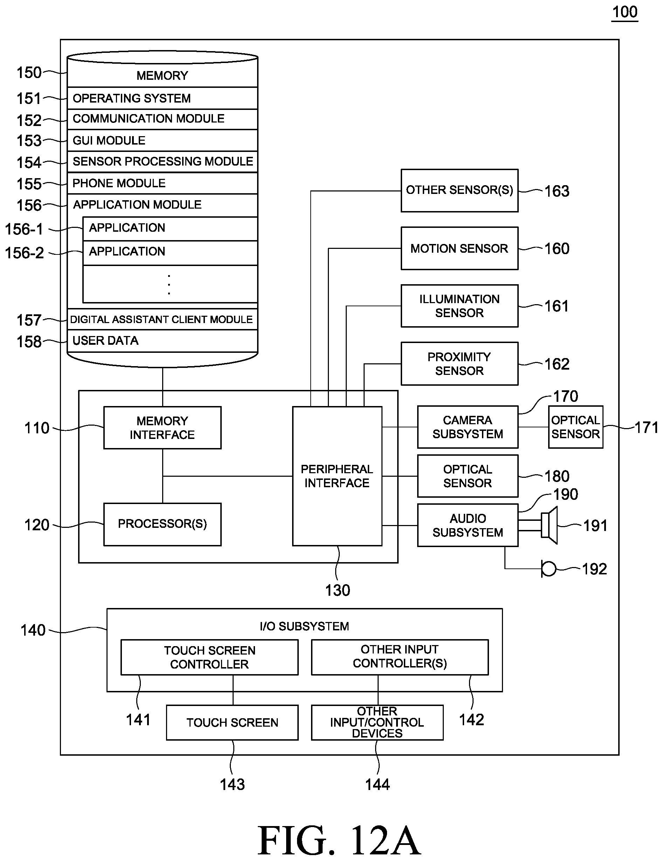

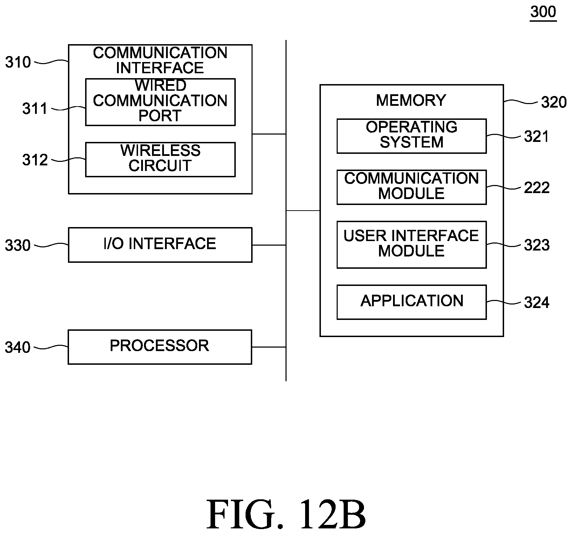

Hereinafter, with reference to FIGS. 11, 12A, and 12B, a guide system for a cross section of an echocardiography using an echocardiography guide device according to an exemplary embodiment of the present invention and an echocardiography guide device will be described.

FIG. 11 illustrates a guide system for a cross section of an echocardiography using an echocardiography guide device according to an exemplary embodiment of the present invention.

FIG. 12A is a block diagram illustrating a configuration of a medical staff device according to an exemplary embodiment of the present invention.

FIG. 12B is a block diagram illustrating a configuration of a guide server according to an exemplary embodiment of the present invention.

First, referring to FIG. 11, the guide system 1000 may be a system configured to provide a guide for echocardiography measurement based on an echocardiographic image of an individual. In this case, the guide system 1000 may be constituted by a medical staff device 100 receiving a guide for echocardiography measurement, an echocardiographic image diagnosis device 200 providing the echocardiographic image, and a guide server 300 generating the guide for echocardiography measurement based on the received echocardiographic image.

First, next, the medical staff device 100 as an electronic device providing a user interface for representing the guide for echocardiography measurement may include at least one of a smartphone, a tablet personal computer (PC), a laptop computer, and/or a PC.

The medical staff device 100 may receive prediction results associated with an electrocardiography cross section for the individual and an angle from the guide server 300, and display the received results through a display unit to be described below.

The guide server 300 may include a general-purpose computer, laptop, and/or data server that perform various operations for determining the guide for echocardiography measurement based on the echocardiographic image provided from the echocardiographic image diagnosis device 200 such as an ultrasound diagnosis device. At this time, the guide server 300 may be a device for accessing a web server providing a web page or a mobile web server providing a mobile website, but is not limited thereto.

More specifically, the guide server 300 receives an echocardiographic image of an individual and a cross section in the image from the echocardiographic image diagnosis device 200, and determines a relative angle between a standard image cross section received in advance and the received cross section to generate a guide for reducing the relative angle based on the relative angle, and finally provide the guide as a graphic interface. At this time, the guide server 300 may determine the relative angle using an Euler angle.

At this time, the provided guide may further include a two-dimensional relative angle for a three-dimensional image determined using the Euler angle.

Further, the provided guide may provide a matching degree between relative angles of the cross section and a standard image.

Accordingly, when diagnosing the heart, an angle at a current position of the probe is compared with the relative angle in the standard image and the cross section to provide a matching degree, so the matching degree may be used as a navigation for acquiring the standard image and the cross section, and it is possible to provide a guideline for highly reliable echocardiography measurement.

The guide server 300 may provide the prediction results to the medical staff device 100.

Information provided from the guide server 300 may be provided to a web page through a web browser installed in the medical staff device 100, or provided in the form of an application or a program. In various exemplary embodiments, the data may be provided in a form that is embedded in a platform in a client-server environment.

Next, with reference to FIGS. 12A and 12B, components of the guide server 300 of the present invention will be described in detail.

First, referring to FIG. 12A, the medical staff device 100 may include a memory interface 110, one or more processors 120, and a peripheral interface 130. Various components within the medical staff device 100 may be connected by one or more communication buses or signal lines.

The memory interface 110 is connected to the memory 150 to transmit various data to the processor 120. Here, the memory 150 may include at least one type of storage medium among flash memory type, hard disk type, multimedia card micro type, card type memory (for example, SD or XD memory, etc.), RAM, SRAM, ROM, EEPROM, PROM, network storage, cloud, and blockchain data.

In various exemplary embodiments, the memory 150 may store at least one of an operating system 151, a communication module 152, a graphical user interface module (GUI) 153, a sensor processing module 154, a phone module 155, and an application module 156. Specifically, the operating system 151 may include instructions for processing basic system services and instructions for performing hardware tasks. The communication module 152 may communicate with at least one of one or more other devices, computers, and servers. The graphical user interface module (GUI) 153 may process a graphical user interface. The sensor processing module 154 may process sensor-related functions (e.g., processing a voice input received using one or more microphones 192). The phone module 155 may process phone-related functions. The application module 156 may perform various functions of the user application, such as electronic messaging, web browsing, media processing, navigation, imaging, and other processing functions. Moreover, the medical staff device 100 may store one or more software applications 156-1 and 156-2 (e.g., a guide application) associated with any one type of service in the memory 150.

In various exemplary embodiments, the memory 150 may store a digital assistant client module 157 (hereinafter, referred to as DA client module), and thus store instructions for performing client-side functions of a digital assistant and various user data 158.

Meanwhile, the DA client module 157 may acquire the user's voice input, text input, touch input, and/or gesture input through various user interfaces (e.g., I/O subsystem 140) provided in the medical staff device 100.

Further, the DA client module 157 may output data in audiovisual and tactile forms. For example, the DA client module 157 may output data consisting of a combination of at least two of voice, sound, notification, text message, menu, graphic, video, animation, and vibration. Moreover, the DA client module 157 may communicate with a digital assistant server (not illustrated) using a communication subsystem 180.

In various exemplary embodiments, the DA client module 157 may collect additional information about a surrounding environment of the medical staff device 100 from various sensors, subsystems, and peripheral devices to construct a context associated with the user input. For example, the DA client module 157 may infer a user's intention by providing context information to a digital assistance server jointly with the user input. Here, the context information that may accompany the user input may include sensor information, such as lighting, ambient noise, ambient temperature, images of the surrounding environment, video, etc. As another example, the context information may include a physical state of the medical staff device 100 (e.g., device orientation, device position, device temperature, power level, speed, acceleration, motion pattern, cellular signal strength, etc.). As yet another example, the context information may include information related to the software state of the medical staff device 100 (e.g., processes running on the medical staff device 100, installed programs, past and current network activity, background services, error logs, resource usage, etc.).

In various exemplary embodiments, the memory 150 may include additional or deleted instructions, and may further include additional components other than those illustrated in FIG. 12A of the medical staff device 100, or may exclude some components.

The processor 120 may control the overall operation of the medical staff device 100 and execute various instructions to implement an interface that provides the guide for echocardiography measurement by running an application or program stored in the memory 150.

The processor 120 may correspond to an operation device such as a central processing unit (CPU) or an application processor (AP). Further, the processor 120 may be implemented in the form of an integrated chip (IC) such as a system on chip (SoC) in which various operation devices such as a neural processing unit (NPU) are integrated.

The peripheral interface 130 is connected to various sensors, subsystems, and peripheral devices to provide data so that the medical staff device 100 may perform various functions. Here, it may be understood that any function performed by the medical staff device 100 is performed by the processor 120.

The peripheral interface 130 may receive data from a motion sensor 160, an illumination sensor (optical sensor) 161, and a proximity sensor 162, and through this, the medical staff device 100 may perform orientation, light, and proximity detection functions. As another example, the peripheral interface 130 may receive data from other sensors 163 (positioning system - GPS receiver, temperature sensor, biometric sensor), thereby enabling the medical staff device 100 to perform functions related to the other sensors 163.

In various exemplary embodiments, the medical staff device 100 may include a camera subsystem 170 connected to the peripheral interface 130, and an optical sensor 171 connected thereto, which may enable the medical staff device 100 to perform various photographic functions, such as taking photographs and recording video clips.

In various exemplary embodiments, the medical staff device 100 may include a communication subsystem 180 connected to the peripheral interface 130. The communication subsystem 180 consists of one or more wired/wireless networks and may include various communication ports, radio frequency transceivers, and optical transceivers.

In various exemplary embodiments, the medical staff device 100 includes an audio subsystem 190 connected to the peripheral interface 130, the audio subsystem 190 includes one or more speakers 191 and one or more microphones 192, such that the medical staff device 100 may perform voice-activated functions, such as speech recognition, voice replication, digital recording, and telephony functions.

In various exemplary embodiments, the medical staff device 100 may include an I/O subsystem 140 connected to the peripheral interface 130. For example, the I/O subsystem 140 may control a touch screen 143 included in the medical staff device 100 via a touch screen controller 141. As an example, the touch screen controller 141 may detect a user's contact and movement or cessation of contact and movement using any one of a plurality of touch sensing technologies, such as capacitive, resistive, infrared, surface acoustic wave technology, proximity sensor array, etc. As another example, the I/O subsystem 140 may control other input/control devices 144 included in the medical staff device 100 via other input controller(s) 142. As an example, the other input controller(s) 142 may control one or more buttons, rocker switches, thumb-wheels, infrared ports, USB ports, and pointer devices such as a stylus.

Next, referring to FIG. 12B, the guide server 300 may include a communication interface 310, a memory 320, an I/O interface 330, and a processor 340, and respective components may communicate with each other through one or more communication buses or signal lines.

The communication interface 310 may be connected to the medical staff device 100 and the echocardiographic image diagnosis device 200 via a wired/wireless communication network to exchange data. For example, the communication interface 310 may receive the echocardiographic image from the echocardiographic image diagnosis device 200, and transmit determined information to the medical staff device 100.

Meanwhile, the communication interface 310 that enables transmission and reception of such data may include a communication port 311 and a wireless circuit 312, and here, the wired communication port 311 may include one or more wired interfaces, for example, Ethernet, universal serial bus (USB), FireWire, etc. Further, the wireless circuit 312 may transmit and receive data with an external device via RF signals or optical signals. Moreover, the wireless communication may use at least one of a plurality of communication standards, protocols and technologies, such as GSM, EDGE, CDMA, TDMA, Bluetooth, Wi-Fi, VoIP, Wi-MAX, or any other suitable communication protocol.

The memory 320 may store various data used in the guide server 300. For example, the memory 320 may store an echocardiographic image, or models trained to determine ultrasound cross sections and relative angles within the echocardiographic image.

In various exemplary embodiments, the memory 320 may include a volatile or nonvolatile storage medium capable of storing various data, instructions, and information. For example, the memory 320 may include at least one type of storage medium among flash memory type, hard disk type, multimedia card micro type, card type memory (for example, SD or XD memory, etc.), RAM, SRAM, ROM, EEPROM, PROM, network storage, cloud, and blockchain data.

In various exemplary embodiments, the memory 320 may store at least one component of an operating system 321, a communication module 322, a user interface module 323, and one or more applications 324.

The operating system 321 (e.g., an embedded operating system such as LINUX, UNIX, MAC OS, WINDOWS, VxWorks, etc.) may include various software components and drivers for controlling and managing general system tasks (e.g., memory management, storage device control, power management, etc.) and may support communication between various hardware, firmware, and software components.

The communication module 323 may support communication with other devices through the communication interface 310. The communication module 320 may include various software components for processing data received by the wired communication port 311 or wireless circuit 312 of the communication interface 310.

The user interface module 323 may receive a user's request or input from a keyboard, touch screen, microphone, etc., through the I/O interface 330 and provide a user interface on a display.

The application 324 may include a program or module configured to be executed by one or more processors 330. Here, the application for providing the guide for echocardiography measurement may be implemented on a server farm.

The I/O interface 330 may connect at least one of an input/output device (not illustrated) of the guide server 300, such as a display, a keyboard, a touch screen, and a microphone, to the user interface module 323. The I/O interface 330 may receive a user input (e.g., a voice input, a keyboard input, a touch input, etc.) together with the user interface module 323, and process an instruction according to the received input.

The processor 340 is connected to the communication interface 310, the memory 320, and the I/O interface 330 to control the overall operation of the guide server 300, and may perform various instructions for the guide through the application or program stored in the memory 320.

The processor 340 may correspond to an operation device such as a central processing unit (CPU) or an application processor (AP). Further, the processor 340 may be implemented in the form of an integrated chip (IC) such as a system on chip (SoC) in which various operation devices are integrated. Alternatively, the processor 340 may include a module for calculating an artificial neural network model, such as a neural processing unit (NPU).

In various exemplary embodiments, the processor 340 may be configured to classify a cross section for the echocardiographic image, determine a relative angle between the standard image cross section received in advance and the received cross section, generate a guide for reducing the relative angle based on the relative angle, and provide the guide as a graphic interface.

In various exemplary embodiments, the processor 340 may be further configured to determine the relative angle using an Euler angle.

In various exemplary embodiments, the processor 340 may be further configured to provide a guide that provides a matching degree between the relative angles of the cross section and the standard image.

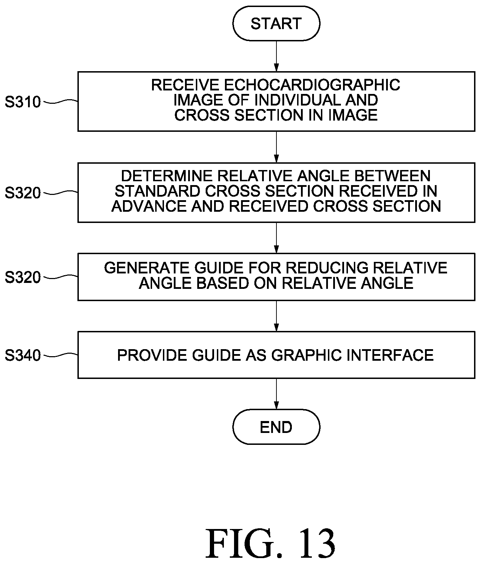

Hereinafter, an echocardiography guide method according to an exemplary embodiment of the present invention will be described in detail with reference to FIG. 13.

FIG. 13 illustrates a procedure of an echocardiography guide method according to an exemplary embodiment of the present invention.

Referring to FIG. 13, a guide procedure according to an exemplary embodiment of the present invention will be described below.

An echocardiographic image of an individual and a cross section in the image may be received, a relative angle between a standard image cross section received in advance and the received cross section may be determined, a guide for reducing the relative angle may be generated based on the relative angle, and the guide may be provided as a graphic interface.

At this time, according to an exemplary embodiment of the present invention, the step of determining the relative angle may be characterized in that an Euler angle is used, and the provided guide may further include a two-dimensional relative angle for a three-dimensional image determined using the Euler angle.

The Euler angle is a three-angle system introduced by Leonhard Euler to represent a direction in which a rigid body is located in three-dimensional space, and a three-dimensional or two-dimensional standard position for each cross section of an echocardiography may be set in advance, and based on this, a relative angle is determined with respect to a current probe position compared to a three-dimensional or two-dimensional baseline.

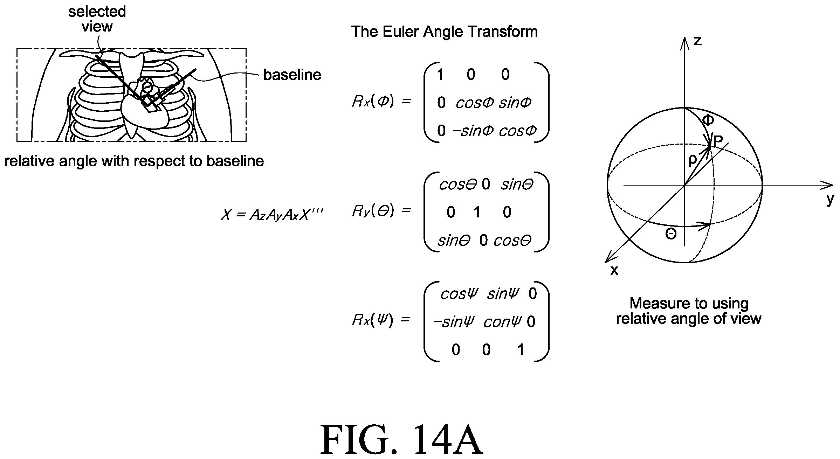

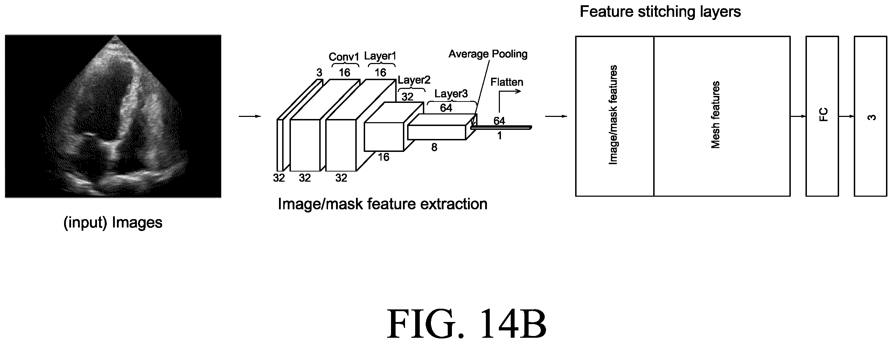

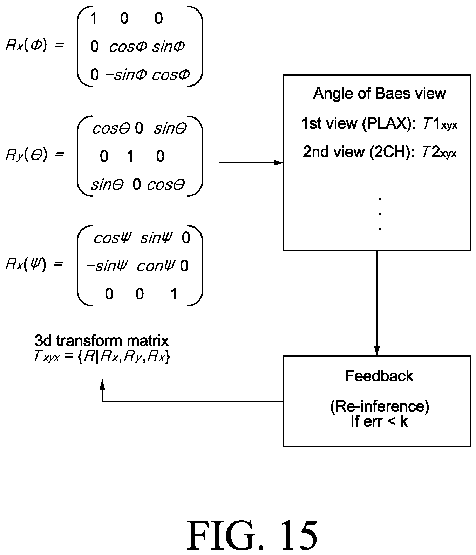

Hereinafter, the echocardiography guide method according to an exemplary embodiment of the present invention will be described in detail with reference to FIGS. 14 and 15.

FIG. 14 is an exemplary diagram illustrating determining a relative angle using an Euler angle according to an exemplary embodiment of the present invention.

FIG. 15 illustrates a procedure for reducing a relative angle according to various exemplary embodiments of the present invention.

First, referring to FIG. 14A, information on a cross section in which an angle of a CT image is labeled is received together with a training ultrasound image to determine a relative angle, and feature extraction for an image and a masking region may be performed, and then the angle may be determined through feature stitching layers. At this time, the angle may be expressed in a transform of a 3D transform matrix, and the feature stitching layers may include a layer for mesh feature extraction.

Next, referring to FIG. 14B, a relative angle between a two-dimensional cross section acquired from a three-dimensional image and a preset baseline is determined using the Euler angle, and referring to FIG. 15, a relative angle between an echocardiographic image for a three-dimensional or two-dimensional standard position and an individual for each cross section of a preset echocardiography, and the cross section in the image is determined to generate and provide a guide including a feedback for reducing the relative angle based on the relative angle, thereby making it possible to more easily acquire an image corresponding to a standard measurement cross section.

The present invention provides a guide for determining a relative angle based on a baseline acquired from a standard measurement image and for reducing the relative angle for a current echocardiographic image acquired by the probe, so that the guide may be utilized as a guide when acquiring a 3D echocardiographic image for acquiring a standard cross section.

Although the exemplary embodiments of the present invention have been described in detail with reference to the accompanying drawings, the present invention is not limited thereto and may be embodied in many different forms without departing from the technical concept of the present invention. Accordingly, the exemplary embodiments disclosed herein are intended to not limit but describe the technical spirit of the present invention but the scope of the technical spirit of the present invention is not limited by the exemplary embodiments. Therefore, it should be appreciated that the aforementioned embodiments are illustrative in all aspects and are not restricted. The protection scope of the present invention should be construed based on the following appended claims and it should be appreciated that the technical spirit included within the scope equivalent to the claims belongs to the scope of the present invention.

**Claims**

1. An echocardiography guide method implemented by a processor, comprising:

   receiving an echocardiographic image of an individual;
   segmenting a cross section of the echocardiographic image into a plurality of cardiac monolayer regions;
   calculating a matching rate for the cross section from which the plurality of cardiac monolayer regions is segmented based on a standard cross section received in advance;
   determining whether to provide a guide based on the matching rate for the cross section; and
   providing the guide for acquiring a cross section having the preset matching rate or higher in response to whether to provide the guide.

2. The echocardiography guide method of claim 1, wherein the segmenting of the cross section of the echocardiographic image into the cardiac monolayer regions further includes
   segmenting each of the plurality of cardiac monolayer regions by using a first prediction model trained to segment the cross section into each of the plurality of cardiac monolayer regions.

3. The echocardiography guide method of claim 1, wherein the calculating of the matching rate for the cross section based on the standard cross section received in advance includes
   calculating the matching rate by using a matching rate calculation model configured to calculate the matching rate by outputting measurement values from the segmented cardiac structure regions and comparing the measurement value with a measurement value determined in the cross section based on the standard cross section received in advance.

4. The echocardiography guide method of claim 1, wherein the plurality of cardiac monolayer regions is at least one selected from a group consisting of right ventricle (RV), left ventricle (LV), aorta (Aorta), left atrium (LA), left ventricle posterior wall (LVPW), and intect ventricular septum (IVS).

5. The echocardiography guide method of claim 2, wherein the first prediction model which is trained to segment the cross section into each of the plurality of cardiac monolayer regions includes a first feature extraction unit configured to input the received echocardiographic image and segment a plurality of regions based on the anatomical structure within the echocardiographic image, and a second feature extraction unit configured to input the received echocardiographic image and predict positions of a plurality of points within the echocardiographic image.

6. The echocardiography guide method of claim 2, wherein the first prediction model is trained to segment the cross section into each of the plurality of cardiac monolayer regions, and is further configured to evaluate the region segmentation result by inputting an entropy, and mask information including an anatomical mask for each region (inferenced chamber mask anatomy) and an area for each region (inferenced chamber mask ratio) with respect to the segmented regions.

7. The echocardiography guide method of claim 3, wherein the matching rate calculation model is configured to calculate a measurement value corresponding to an area and a size of each of the segmented cardiac monolayer regions by inputting a plurality of points in the echocardiographic image determined by the first prediction model, and further configured to calculate the matching rate by comparing the measurement value determined from the cross section based on the standard cross section received in advance.

8. The echocardiography guide method of claim 7, wherein the matching rate calculation model is further configured to calculate the matching rate by evaluating measurement values measured in the segmented cardiac monolayer regions by inputting data from an entropy meter, a capacity meter, and a connectivity meter, and comparing the measurement value determined in the cross section based on the standard cross section received in advance.

9. An echocardiography guide device comprising:

   a communication unit configured to receive an echocardiographic image of an individual; and
   a processor functionally connected to the communication unit,
   wherein the processor is configured to
   segment a cross section of the echocardiographic image into a plurality of cardiac monolayer regions,
   calculate a matching rate for the cross section from which the plurality of cardiac monolayer regions is segmented based on a standard cross section received in advance,
determine whether to provide a guide based on the matching rate for the cross section, and
provide the guide for acquiring a cross section having the preset matching rate or higher in response to whether to provide the guide.

10. The echocardiography guide device of claim 9, wherein the processor is further configured to segment each of the plurality of cardiac monolayer regions by using a first prediction model trained to segment the cross section into each of the plurality of cardiac monolayer regions.

11. The echocardiography guide device of claim 9, wherein the processor is further configured to calculate the matching rate by using a matching rate calculation model configured to calculate the matching rate by outputting measurement values from the segmented cardiac structure regions and comparing the measurement value with a measurement value determined in the cross section based on the standard cross section received in advance.

12. The echocardiography guide device of claim 9, wherein the plurality of cardiac monolayer regions consists of right ventricle (RV), left ventricle (LV), the aorta (Aorta), left atrium (LA), left ventricle posterior wall (LVPW), and intect ventricular septum (IVS).

13. The echocardiography guide device of claim 10, wherein the first prediction model which is trained to segment the cross section into each of the plurality of cardiac monolayer regions includes a first feature extraction unit configured to input the received echocardiographic image and segment a plurality of regions based on the anatomical structure within the echocardiographic image, and a second feature extraction unit configured to input the received echocardiographic image and predict positions of a plurality of points within the echocardiographic image.

14. The echocardiography guide device of claim 10, wherein the first prediction model is trained to segment the cross section into each of the plurality of cardiac monolayer regions, and is further configured to evaluate the region segmentation result by inputting an entropy, and mask information including an anatomical mask for each region (inferenced chamber mask anatomy) and an area for each region (inferenced chamber mask ratio) with respect to the segmented regions.

15. The echocardiography guide device of claim 11, wherein the matching rate calculation model is configured to calculate a measurement value corresponding to an area and a size of each of the segmented cardiac monolayer regions by inputting a plurality of points in the echocardiographic image determined by the first prediction model, and further configured to calculate the matching rate by comparing the measurement value determined from the cross section based on the standard cross section received in advance.

16. The echocardiography guide device of claim 15, wherein the matching rate calculation model is further configured to calculate the matching rate by evaluating measurement values measured in the segmented cardiac monolayer regions by inputting data from an entropy meter, a capacity meter, and a connectivity meter, and comparing the measurement value determined in the cross section based on the standard cross section received in advance.

17. The echocardiography guide device of claim 9, wherein a visualization function such as a navigation of a two-dimensional image according to a position and a direction in which a probe moves in a virtual three-dimensional structure for an entire heart is included with respect to movement of the probe.

18. An echocardiography guide method implemented by a processor, comprising:

receiving an echocardiographic image of an individual and a cross section in the image;
determining a relative angle between a standard image cross section received in advance and the received cross section;
generating a guide for reducing the relative angle based on the relative angle; and
providing the guide as a graphic interface.

19. The echocardiography guide method of claim 18, wherein in the determining of the relative angle, an Euler angle is used.

20. The echocardiography guide method of claim 18, wherein the provided guide further includes a two-dimensional relative angle for a three-dimensional image determined using the Euler angle.

21. The echocardiography guide method of claim 20, wherein the provided guide provides a matching degree between relative angles of the cross section and a standard image.

22. An echocardiography guide device comprising:

a communication unit configured to receive an echocardiographic image of an individual and a cross section in the image; and
a processor functionally connected to the communication unit,
wherein the processor is configured to
classify a cross section for the echocardiographic image,
determine a relative angle between a standard image cross section received in advance and the received cross section,
generate a guide for reducing the relative angle based on the relative angle, and
provide the guide as a graphic interface.

23. The echocardiography guide device of claim 22, wherein the processor is further configured to determine the relative angle using an Euler angle.

24. The echocardiography guide device of claim 22, wherein the processor is further configured to provide further include a two-dimensional relative angle for a three-dimensional image determined using the Euler angle.

25. The echocardiography guide device of claim 24, wherein the provided guide provides a matching degree between relative angles of the cross section and a standard image.

1000

FIG. 1

FIG. 2A

300

310 — COMMUNICATION INTERFACE

311 — WIRED COMMUNICATION PORT

312 — WIRELESS CIRCUIT

330 — I/O INTERFACE

340 — PROCESSOR

MEMORY — 320

OPERATING SYSTEM — 321

COMMUNICATION MODULE — 222

USER INTERFACE MODULE — 323

APPLICATION — 324

# FIG. 2B

START

S310 — RECEIVE ECHOCARDIOGRAPHIC
IMAGE OF INDIVIDUAL

S320 — SEGMENT CROSS SECTION OF
ECHOCARDIOGRAPHIC IMAGE INTO PLURALITY OF
CARDIAC MONOLAYER REGIONS

S330 — CALCULATE MATCHING RATE FOR CROSS
SECTION FROM WHICH PLURALITY OF CARDIAC
MONOLAYER REGIONS IS SEGMENTED BASED ON
STANDARD CROSS SECTION RECEIVED IN
ADVANCE

S340 — DETERMINE WHETHER TO PROVIDE GUIDE BASED
ON MATCHING RATE OF CROSS SECTION

S350 — IN RESPONSE TO WHETHER TO PROVIDE GUIDE,
PROVIDE GUIDE FOR ACQUIRING CROSS SECTION
HAVING PRESET MATCHING RATE OR HIGHER

END

# FIG. 3

RV
$(x_5, y_5)$

IVS
$(x_2, y_2)$

Aorta
$(x_1, y_1)$

LV
$(x_4, y_4)$

LV-pw
$(x_6, y_6)$

LA
$(x_3, y_3)$

FIG. 4A

11%

19%

18%

5%

15%

32%

| aorta | ivs | la capacity |
| lv capacity | rvc | lv-pw |

# FIG. 4B

FIG. 5A

FIG. 5B

FIG. 6A

FIG. 6B

FIG. 6C

FIG. 7

FIG. 8

EP 4 512 343 A2

FIG. 9

FIG. 10

FIG. 11

100

| | |
|---|---|
| 150 | MEMORY |
| 151 | OPERATING SYSTEM |
| 152 | COMMUNICATION MODULE |
| 153 | GUI MODULE |
| 154 | SENSOR PROCESSING MODULE |
| 155 | PHONE MODULE |
| 156 | APPLICATION MODULE |
| 156-1 | APPLICATION |
| 156-2 | APPLICATION |
| 157 | DIGITAL ASSISTANT CLIENT MODULE |
| 158 | USER DATA |

OTHER SENSOR(S) ~163

MOTION SENSOR ~160

ILLUMINATION SENSOR ~161

PROXIMITY SENSOR ~162

110 MEMORY INTERFACE

120 PROCESSOR(S)

PERIPHERAL INTERFACE

130

170
CAMERA SUBSYSTEM — OPTICAL SENSOR ~171

OPTICAL SENSOR ~180

190
AUDIO SUBSYSTEM ~191
~192

140 I/O SUBSYSTEM

TOUCH SCREEN CONTROLLER

OTHER INPUT CONTROLLER(S)

141 TOUCH SCREEN

OTHER INPUT/CONTROL DEVICES 142

143 144

# FIG. 12A

EP 4 512 343 A2

300

310 — COMMUNICATION INTERFACE

311 — WIRED COMMUNICATION PORT

312 — WIRELESS CIRCUIT

MEMORY — 320

OPERATING SYSTEM — 321

COMMUNICATION MODULE — 222

USER INTERFACE MODULE — 323

APPLICATION — 324

330 — I/O INTERFACE

340 — PROCESSOR

# FIG. 12B

38

START

↓

S310 — RECEIVE ECHOCARDIOGRAPHIC
IMAGE OF INDIVIDUAL AND
CROSS SECTION IN IMAGE

↓

S320 — DETERMINE RELATIVE ANGLE BETWEEN
STANDARD CROSS SECTION RECEIVED IN
ADVANCE AND RECEIVED CROSS SECTION

↓

S320 — GENERATE GUIDE FOR REDUCING RELATIVE
ANGLE BASED ON RELATIVE ANGLE

↓

S340 — PROVIDE GUIDE AS GRAPHIC INTERFACE

↓

END

# FIG. 13

selected
view

baseline

relative angle with respect to baseline

The Euler Angle Transform

$$R_x(\Phi) = \begin{pmatrix} 1 & 0 & 0 \\ 0 & cos\Phi & sin\Phi \\ 0 & -sin\Phi & cos\Phi \end{pmatrix}$$

$$X = A_z A_y A_x X'''$$

$$R_y(\Theta) = \begin{pmatrix} cos\Theta & 0 & sin\Theta \\ 0 & 1 & 0 \\ sin\Theta & 0 & cos\Theta \end{pmatrix}$$

$$R_x(\Psi) = \begin{pmatrix} cos\Psi & sin\Psi & 0 \\ -sin\Psi & con\Psi & 0 \\ 0 & 0 & 1 \end{pmatrix}$$

Measure to using
relative angle of view

FIG. 14A

Feature stitching layers

Image/mask feature extraction

(input) Images

FIG. 14B

EP 4 512 343 A2

$$R_x(\Phi) = \begin{pmatrix} 1 & 0 & 0 \\ 0 & cos\Phi & sin\Phi \\ 0 & -sin\Phi & cos\Phi \end{pmatrix}$$

$$R_y(\Theta) = \begin{pmatrix} cos\Theta & 0 & sin\Theta \\ 0 & 1 & 0 \\ sin\Theta & 0 & cos\Theta \end{pmatrix}$$

$$R_x(\Psi) = \begin{pmatrix} cos\Psi & sin\Psi & 0 \\ -sin\Psi & con\Psi & 0 \\ 0 & 0 & 1 \end{pmatrix}$$

3d transform matrix
$T_{xyx} = \{R | R_x, R_y, R_x\}$

Angle of Baes view

1st view (PLAX): $T1_{xyx}$

2nd view (2CH): $T2_{xyx}$

.
.
.

Feedback

(Re-inference)
If err < k

# FIG. 15